# EUROPÄISCHE PATENTANMELDUNG

(11) **EP 0 748 811 A1**
(43) Veröffentlichungstag der Anmeldung: **18.12.1996**
(21) Anmeldenummer: 96108972.9
(22) Anmeldetag: 05.06.1996
(51) Int. Cl.: C07F 9/50, C07F 9/6568

(54) **Verfahren zur Herstellung von Hydroxybiarylphosphanen und neue Verbindungen aus dieser Stoffgruppe**

(30) Priorität: 12.06.1995 DE 19521340
(71) Anmelder: HOECHST AKTIENGESELLSCHAFT, 65929 Frankfurt am Main (DE)
(72) Erfinder: Regnat, Dieter, Dr., 65817 Eppstein (DE); Kleiner, Hans-Jerg, Dr., 61476 Kronberg (DE)

(57) **Zusammenfassung**

Die vorliegende Erfindung betrifft ein Verfahren zur Herstellung von Hydroxybiarylphosphanen der allgemeinen Formel 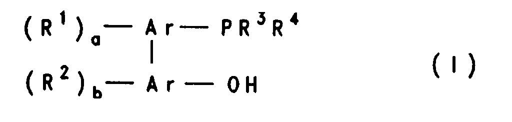 worin R¹, R², a, b, Ar-Ar, R³ und R⁴ die nachfolgende Bedeutung haben, indem man ein Oxaphosphorin der allgemeinen Formel 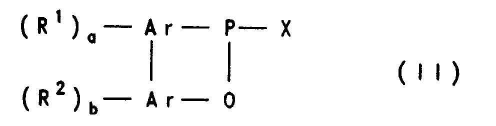 worin Ar-Ar für Biphenyl, 1-Phenylnaphthyl oder 1,1'-Binaphthyl steht, R¹ und R² gleich oder verschieden sind und für F, Cl, Alkyl oder Alkoxy mit jeweils 1 bis 8 Kohlenstoffatomen oder substituiertes oder unsubstituiertes Aryl stehen, a und b gleich oder verschieden sind und für eine ganze Zahl von 0 bis 4 stehen, X jeweils für Cl oder Br steht und die Ar-P und die Ar-O-Bindung jeweils in ortho-Stellung zur Ar-Ar-Bindung angeordnet ist, mit einer Verbindung R³-Me und R⁴-Me, worin R³ und R⁴ gleich oder verschieden sind und für Alkyl mit 1 bis 8 Kohlenstoffatomen Cycloalkyl mit 4 bis 10 Kohlenstoffatomen, Alkaryl mit 7 bis 9 Kohlenstoffatomen, Ar³-(R⁵)ₙ, wobei Ar³ ein Arylrest mit 6 bis 10 Kohlenstoffatomen, R⁵ Alkyl oder Alkoxy mit jeweils 1 bis 8 Kohlenstoffatomen, F, Cl, CF₃, Dialkylamino mit insgesamt 2 bis 8 Kohlenstoffatomen oder - CH₂N(Alkyl)₂, mit 1 bis 3 Kohlenstoffatomen je Alkyl ist und n eine ganze Zahl von 0 bis 5 darstellt, stehen, oder mit einer Verbindung Me-R³-R⁴-Me, worin - R³-R⁴- eine Kette von 3 bis 5 Gliedern bildet, an der gegebenenfalls ein oder zwei aromatische Ringe oder Ringsysteme mit 6 bis 10 Kohlenstoffatomen anelliert sind, Me für ein einwertiges Metall oder für ein Äquivalent eines mehrwertigen Metalles steht, in Anwesenheit eines Lösungsmittels bei einer Temperatur von -40 bis 160°C umsetzt, anschließend mit Wasser und gegebenenfalls einem wasserunlöslichen organischen Solvens versetzt und die wäßrige Phase von der organischen Phase trennt.
Die Erfindung betrifft ferner Verbindungen aus der Reihe der Hydroxybisarylphosphane.

## Beschreibung

Die vorliegende Erfindung betrifft ein gegenüber dem Stand der Technik verbessertes Verfahren zur Herstellung von Hydroxybiarylphosphanen und neue Verbindungen aus dieser Stoffgruppe.

Hydroxybiarylphosphane stellen wichtige Zwischenprodukte, beispielsweise für die Synthese von Phosphinphosphit-Liganden der allgmeinen Formel (A) 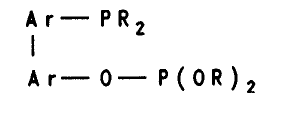 dar.
Die vorstehend genannten Phosphinphosphit-Liganden finden bei der enantioselektiven Hydroformylierung Verwendung (EP 0 614 901, EP 0 614 902, EP 0 614 903, EP 0 614 870). Mittels der enantioselektiven Hydroformylierung lassen sich durch Umsetzung eines geeigneten Olefins mit Kohlenmonoxid und Wasserstoff beispielsweise optisch aktive Aldehyde herstellen.

Die Herstellung der als Zwischenprodukte für die Herstellung der Phosphinphosphit-Liganden benötigten Hydroxybiarylphosphane ist in der EP 0 614 903 sowie in Tetrahedron Letters, 31, 6321 (1990) und J. Org. Chem. 58, 1945 (1993) näher beschrieben.

Sie geht von Dihydroxybiarylverbindungen aus und verläuft, durch die nachstehenden Reaktionsgleichungen näher beschrieben, über nicht weniger als 4 Stufen. Diese Reaktionsstufen sind zum Teil kostspielig und darüber hinaus mit einem hohen Aufwand für die erforderlichen Reinigungsschritte verbunden.

Die Umsetzung verläuft über die folgenden 4 Stufen:
1. Stufe 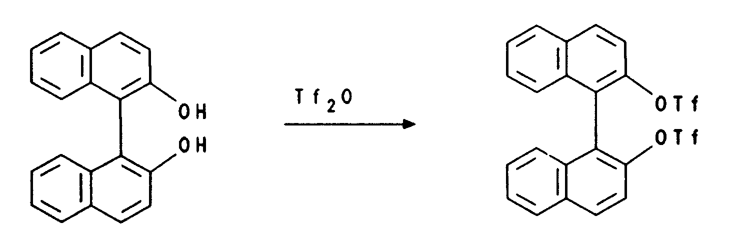 Tf₂O: Trifluormethylsulfonsäureanhydrid
2. Stufe 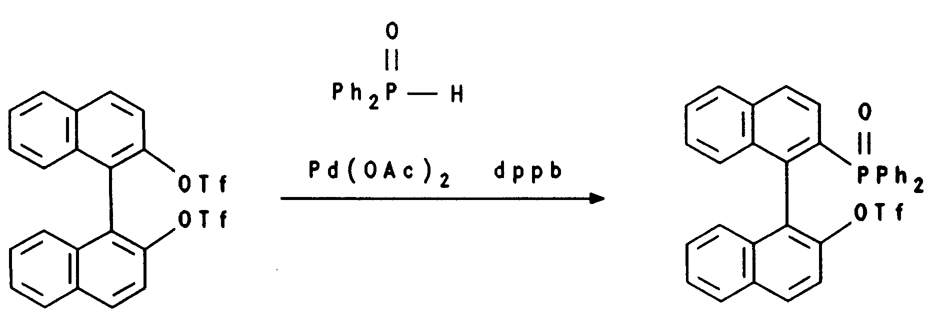 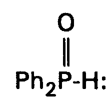 Diphenylphosphanoxid
   Pd(OAc)₂: Pd acetat
   dppb: 1,4-Bis(diphenylphosphino)butan
3. Stufe 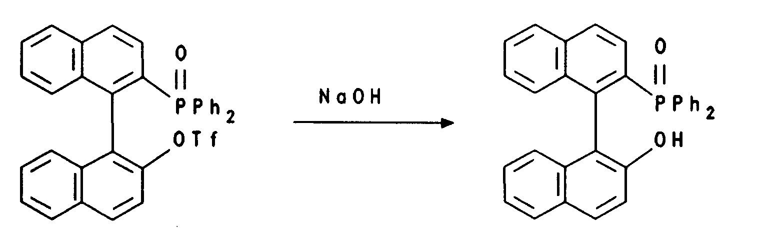
4. Stufe 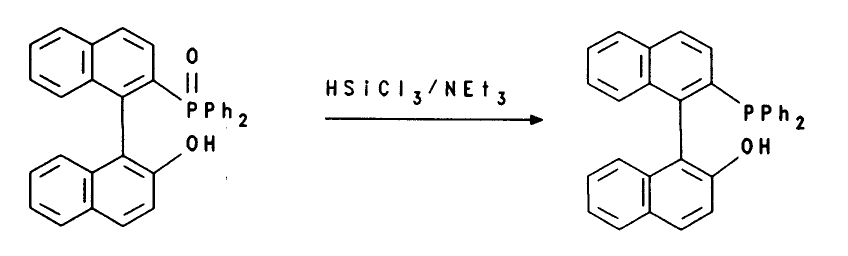 HSiCl₃: Trichlorsilan
   NEt₃: Triethylamin

Ein weiterer Nachteil dieses Verfahrens besteht darin, daß man in der 2. Stufe mit einem disubstituierten Phosphanoxid, nämlich Diphenylphosphanoxid, arbeiten muß. Geeignete disubstituierte Phosphanoxide stehen jedoch nur in begrenzter Zahl zur Verfügung. Dies grenzt die Möglichkeiten dieser Synthese erheblich ein.

Ein weiterer entscheidender Nachteil ist, daß das Diphenylphosphanoxid wahllos mit einem der beiden TfO-Reste reagiert. Diese Umsetzung ist daher nicht geeignet, gezielt einen der beiden TfO-Reste durch den Diphenylphosphanoxidrest zu substituieren. Es bildet sich stets ein Gemisch beider Substitutionsprodukte. Dies ist insbesondere bei der Verwendung von unsymmetrischen Dihydroxy-biarylverbindungen von Nachteil, da sich Stoffgemische bilden, die sich nicht mehr oder nur mit einem unverhältnismäßig hohen Aufwand in die einzelnen Komponenten trennen lassen.

Dieses Verfahren eignet sich deshalb hautpsächlich nur für die Umsetzung symmetrischer Dihydroxybiarylverbindungen, in denen die beiden Hydroxygruppen gleich im Sinne von ununterscheidbar sind. Hierbei kommt es nämlich nicht darauf an, welche der beiden Hydroxygruppen bzw. TfO-Reste gegen das Diphenylphosphanoxid ausgetauscht wird, da ein Austausch der einen Hydroxygruppe bzw. des einen TfO-Restes ebenso wie ein Austausch der anderen Hydroxygruppe bzw. des anderen TfO-Restes immer zum selben Endprodukt führt.

Daher besteht ein lebhaftes Interesse ein Verfahren zur Herstellung von Hydroxybiarylphosphanen bereitzustellen, das die vorstehend beschriebenen Nachteile nicht aufweist, sich ausgehend von vergleichsweise einfach zugänglichen Ausgangstoffen ohne großen Aufwand technisch realisieren läßt, nicht von Dihydroxybiarylverbindungen ausgeht und demzufolge auch nicht auf die Umsetzung symmetrischer Dihydroxybiarylverbindungen beschränkt ist.

Gelöst wird diese Aufgabe durch ein Verfahren zur Herstellung von Hydroxybiarylphosphanen der allgemeinen Formel 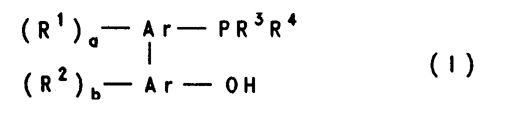 worin R¹, R², a, b, Ar-Ar, R³ und R⁴ die nachfolgende Bedeutung haben. Es ist dadurch gekennzeichnet, daß man ein Oxaphosphorin der allgemeinen Formel 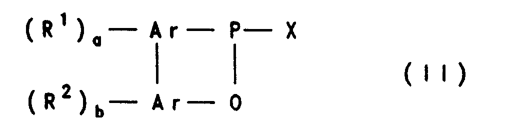 worin Ar-Ar für Biphenyl, 1-Phenylnaphthyl oder 1,1'-Binaphthyl steht, R¹ und R² gleich oder verschieden sind und für F, Cl, Alkyl oder Alkoxy mit jeweils 1 bis 8 Kohlenstoffatomen oder substituiertes oder unsubstituiertes Aryl stehen, a und b gleich oder verschieden sind und für eine ganze Zahl von 0 bis 4 stehen, X jeweils für Cl oder Br steht und die Ar-P und die Ar-O-Bindung jeweils in ortho-Stellung zur Ar-Ar-Bindung angeordnet ist, mit einer Verbindung R³-Me und R⁴-Me, worin R³ und R⁴ gleich oder verschieden sind und für Alkyl mit 1 bis 8 Kohlenstoffatomen, Cycloalkyl mit 4 bis 10 Kohlenstoffatomen, Alkaryl mit 7 bis 9 Kohlenstoffatomen, Ar³-(R⁵)ₙ, wobei Ar³ ein Arylrest mit 6 bis 10 Kohlenstoffatomen, R⁵ Alkyl oder Alkoxy mit jeweils 1 bis 8 Kohlenstoffatomen, F, Cl, CF₃, Dialkylamino mit insgesamt 2 bis 8 Kohlenstoffatomen oder - CH₂N(Alkyl)₂ mit 1 bis 3 Kohlenstoffatomen je Alkyl ist und n eine ganze Zahl von 0 bis 5 darstellt, stehen, oder mit einer Verbindung Me-R³-R⁴-Me, worin - R³-R⁴- eine Kette von 3 bis 5 Gliedern bildet, an der gegebenenfalls ein oder zwei aromatische Ringe oder Ringsysteme mit 6 bis 10 Kohlenstoffatomen anelliert sind, Me für ein einwertiges Metall oder für ein Äquivalent eines mehrwertigen Metalles steht, in Anwesenheit eines Lösungsmittels bei einer Temperatur von -40 bis 160°C umsetzt, anschließend mit Wasser und gegebenenfalls einem wasserunlöslichen organischen Solvens versetzt und die wäßrige Phase von der organischen Phase trennt.

Die Umsetzung verläuft, beispielhaft durch die nachstehende Gleichung beschrieben, unter Aufspaltung des P-Ringes im Oxaphosphorin (II) ab:

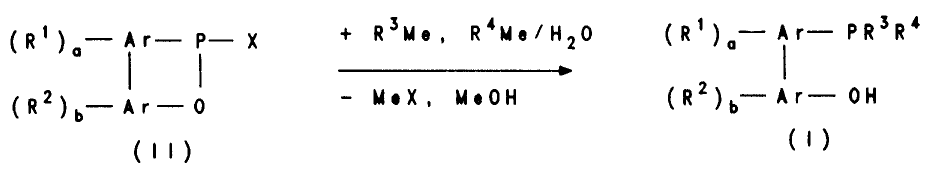

Es ist als sehr überraschend anzusehen, daß es mit Hilfe des erfindungsgemäßen Verfahrens möglich ist, den das Phosphoratom enthaltenden Ring im Oxaphosphorin der Formel (II) mit sehr hoher Selektivität zu öffnen, wobei nicht nur die Ar-Ar-Bindung, sondern zugleich auch die Ar-P-Bindung unverändert erhalten bleibt.

Dadurch ist sichergestellt, daß auch bei Einsatz unsymmetrischer Oxaphosphorine stets ein einheitliches Hydroxybiarylphosphan und nicht verschiedene Hydroxybiarylphosphane und weitere Neben- und Abbauprodukte enthaltendes Gemisch gebildet wird.

Darüber hinaus gestattet es das erfindungsgemäße Verfahren, das Phosphoratom gezielt entweder mit zwei gleichen Resten oder mit zwei verschiedenen Resten zu substituieren.

Das erfindungsgemäße Verfahren geht ferner von vergleichsweise leicht zugänglichen Oxaphosphorinen der Formel (II) aus, deren Herstellung Gegenstand einer am gleichen Tag wie die vorliegende Patentanmeldung eingereichten deutschen Patentanmeldung (Aktenzeichen: 195 21 339.4) ist.

Ein weiterer Vorteil des erfindungsgemäßen Verfahrens besteht darin, daß es nicht erforderlich ist, zwischen den einzelnen Arbeitsschritten eines der Zwischenprodukte zu isolieren, um dieses nachfolgend weiter zu verarbeiten.

Um zu verdeutlichen, welche Oxaphosphorine der Formel (II) eingesetzt werden können, seien die nachfolgenden Verbindungen der Formeln (II), (IIIa), (IV), (IVa) und (V) erwähnt, ohne jedoch hierbei einen Anspruch auf Vollständigkeit der Aufzählung zu erheben.

Die Oxaphosphorine der Formel (III) 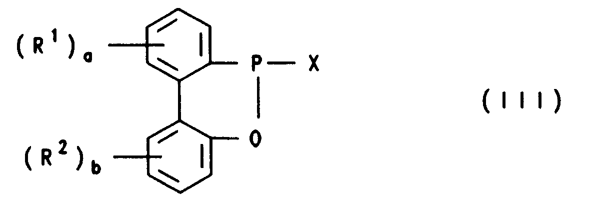 leiten sich vom 6-Chlor-6H-dibenz[c,e][1,2]oxaphosphorin oder 6-Brom-6H-dibenz[c,e][1,2]oxaphosphorin ab. Bei dem Oxaphosphorin der Formel (IIIa) 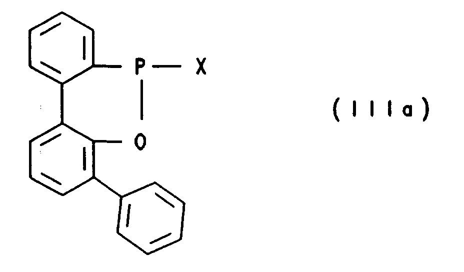 handelt es sich um das 6-Chlor-6H-4-phenyl-dibenz[c,e][1,2]oxaphosphorin oder 6-Brom-6H-4-phenyl-dibenz[c,e][1,2]oxaphosphorin. Die einen 1-Phenylnaphthylrest als Ar-Ar enthaltenden Oxaphosphorine der Formel (IV) 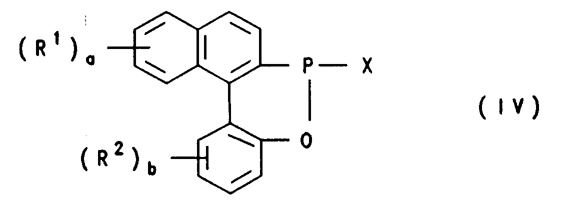 leiten sich vom 6-Chlor-6H-benzo[e]naphth[2,1-c][1,2]oxaphosphorin oder 6-Brom-6H-benzo[e]naphth[2,1-c][1,2]oxaphosphorin her und die ebenfalls einen 1-Phenylnaphthylrest als Ar-Ar enthaltenden Oxaphosphorine der Formel (IVa) 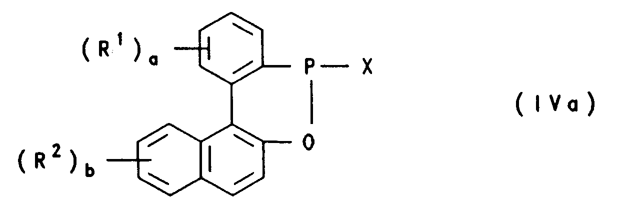 leiten sich vom 5-Chlor-5H-benzo[c]naphth[1,2-c][1,2]oxaphosphorin oder 5-Brom-5H-benzo[c]naphth[1,2-c][1,2]oxaphosphorin ab.

Die einen Binaphthylrest als Ar-Ar enthaltenden Oxaphosphorine der Formel (V) 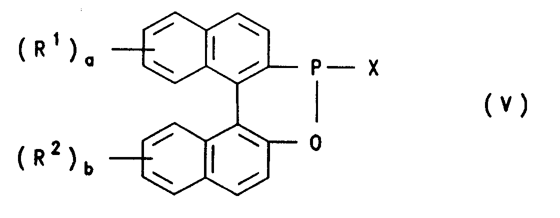 leiten sich vom 3-Chlor-3H-dinaphtho[2,1-c:1',2'-e][1,2]oxaphosphorin oder 3-Brom-3H-dinaphtho[2,1-c:1',2'-e][1,2]oxaphosphorin ab.

Es versteht sich von selbst, daß in den vorstehend genannten Formeln R¹, R², a und b die bereits erwähnte Bedeutung haben.

Von besonderem Interesse sind Oxaphosphorine der allgemeinen Formel (II), in denen R¹ und R² die vorstehend genannte Bedeutung besitzen und a für 0 und b für 1 oder a für 1 und b für 0 steht. Diese Oxaphosphorine sind relativ leicht zugänglich, da sie lediglich einen Rest R¹ und R² aufweisen, und zugleich sind sie unsymmetrisch. Als besonders bevorzugt sind Oxaphosphorine der allgemeinen Formel (II) anzusehen, in denen a und b jeweils für 0 steht.

Das erfindungsgemäße Verfahren läßt sich mit gutem Erfolg durchführen, indem man ein Oxaphosphorin:der Formel (II) einsetzt, worin R¹ und R² gleich oder verschieden sind und für F, Cl, Alkyl oder Alkoxy mit jeweils 1 bis 4 Kohlenstoffatomen oder substituiertes oder unsubstituiertes Phenyl stehen, a und b gleich oder verschieden sind und für eine ganze Zahl von 0 bis 2 stehen, insbesondere a für 0 und b für 1 oder a für 1 und b für 0 steht, und indem man insbesondere ein Oxaphosphorin der allgemeinen Formel (II) einsetzt, worin R¹ und R² gleich oder verschieden sind und für F, Cl, Alkyl oder Alkoxy mit jeweils 1 bis 4 Kohlenstoffatomen stehen, a und b gleich oder verschieden sind und für 0 oder 1 stehen, insbesondere a und b verschieden sind und für 0 oder 1 stehen.

Nach einer Verfahrensvariante setzt man ein Oxaphosphorin der Formel (II) ein, worin Ar-Ar für 1-Phenylnaphthyl steht und die Ar-P-Bindung am Phenyring des 1-Phenylnaphthyls oder am Naphthylring des 1-Phenylnaphthyls angeordnet ist. Diese Oxaphosphorine können, wie bereits durch die Verbindungen der Formel (IV) und (IVa) erläutert, durch (R¹)ₐ und (R¹)_{b} substituiert sein, wobei wegen ihrer relativ guten Verfügbarkeit derartige Oxaphosphorine, in denen a für 0 und b für 1 oder a für 1 und b für Null oder in denen a und b jeweils für Null steht, ein besonderes Interesse verdienen.

Als Verbindungen R³-Me und/oder R⁴-Me oder Me-R³-R⁴-Me, worin R³-R⁴ miteinander verbunden sind und eine Kette mit 3 bis 5 Kohlenstoffatomen bilden und die Kette substituiert oder unsubstituiert sein kann, insbesondere an die Kette ein oder zwei aromatische Ringe oder Ringsysteme mit jeweils 6 bis 10 Kohlenstoffatomen anelliert sein können, lassen sich generell Organometallverbindungen einsetzen. Me steht beispielsweise für MgX, wobei X für Chlor, Brom oder Jod, insbesondere Chlor oder Brom steht, oder für ein Alkalimetall, beispielsweise Lithium, Natrium oder Kalium, insbesondere für Natrium oder Lithium, bevorzugt Lithium.

Man führt die Umsetzung in Anwesenheit eines Lösungsmittels durch. Es empfiehlt sich, als Lösungsmittel ein dipolar aprotisches Lösungsmittel oder ein unpolares Lösungsmittel oder eine Mischung dieser Lösungsmittel zu verwenden.
In diesem Zusammenhang sei darauf hingewiesen, daß Organometallverbindungen zumeist in bestimmten Lösungsmitteln gehandhabt werden. Grignard-Verbindungen gelangen häufig gelöst oder suspendiert in einem Ether zum Einsatz.
Man kann - ohne Anspruch auf Vollständigkeit zu erheben - als Lösungsmittel einen Ether oder ein Ethergemisch, beispielsweise Dialkylether mit 2 bis 4 Kohlenstoffatomen je Alkyl, Tetrahydrofuran, Dioxan, Toluol, ortho-Xylol, meta-Xylol, para-Xylol, ein Gemisch isomerer Xylole, Mesitylen, Ethylbenzol oder Gemische dieser Lösungsmittel einsetzen.

Die Umsetzung verläuft vermutlich in zwei Stufen, wobei zunächst ein Äquivalent der Organometallverbindung mit dem Oxaphosphorin reagiert. Hierbei wird wahrscheinlich das am P-Atom befindliche X im Oxaphosphorin der Formel (II) substituiert.

In einer nachfolgenden Stufe reagiert ein weiteres Äquivalent der Organometallverbindungen mit dem bereits substituierten P-Atom des Oxaphosphorins wobei der Ring wahrscheinlich durch Spaltung der P-O-Bindung geöffnet wird.

Unter Ausnutzung dieser Reaktionsfolge kann man das Oxaphosphorin der Formel (II) zunächst mit der Verbindung R³-Me und anschließend mit der Verbindung R⁴-Me umsetzen oder aber auch umgekehrt das Oxaphosphorin der Formel (II) zunächst mit der Verbindung R⁴-Me und anschließend mit der Verbindung R³-Me zur Reaktion bringen.
Dadurch erhält man auf einfache Art und Weise Hydroxybiarylphosphane der Formel I, deren P-Atom, bedingt durch die Substitution durch zwei unterschiedliche Reste R³ und R⁴, insgesamt drei verschiedene Reste aufweist, nämlich den Hydroxybiarylrest, den Rest R³ und den Rest R⁴.

Diese Reaktionsfolge läßt sich selbstverständlich auch auf die Umsetzung der Verbindung Me-R³-R⁴-Me anwenden, wobei das Phosphoratom in einen Ring eingebaut wird.

In den meisten Fällen genügt es, die Umsetzung bei einer Temperatur von 40 bis 160°C, insbesondere 60 bis 120°C durchzuführen. Dies betrifft die gesamte Reaktionsfolge, also beide Stufen.
Man kann aber auch die Umsetzung in einer ersten Stufe bei -40 bis 80, insbesondere -20 bis 60, bevorzugt -10 bis 50°C und anschließend in einer zweiten Stufe bei 20 bis 160, insbesondere 40 bis 140, bevorzugt 60 bis 120°C ablaufen lassen.

Die Reaktionsbedingungen hängen in gewissem Umfang auch von der Art der verwendeten Organometallverbindung ab.

Organometallverbindungen mit einer hohen Reaktivität, beispielsweise Alkylgrignard-Verbindungen oder Alkalimetallalkyle, lassen sich bereits bei recht niedrigen Temperaturen umsetzen, während weniger reaktive Organometallverbindungen, beispielsweise Arylgrignard-Verbindungen, höhere Reaktionstemperaturen bedingen.
Die Reaktionsbedingungen hängen aber auch in gewissem Umfange von der Art des verwendeten Oxaphosphorins ab.
Oxaphosphorine der Formel (II) mit erhöhter Reaktivität lassen sich bei niedrigeren Temperaturen umsetzen. Hierzu dürften Oxaphosporine zählen, deren das P-Atom enthaltende Ring, beispielsweise durch Verdrehung der beiden Arylreste gegeneinander um die Ar-Ar-Bindung, unter Spannung steht. Oxaphosphorine, deren das P-Atom enthaltender Ring keine oder nur eine geringe Spannung aufweist, oder Oxaphosphorine, deren P-Atom infolge sperriger Substituenten sterisch gehindert ist und einem Angriff der Organometallverbindung nicht frei zugänglich ist, können hingegen eine Reaktion bei höheren Temperaturen erforderlich machen.

Üblicherweise legt man eine Lösung des Oxaphosphorins der Formel (II) vor, wobei eines der vorstehend genannten Lösungsmittel verwendet werden kann. Zu dieser Lösung tropft man unter Rühren eine Lösung oder Suspension der Organometallverbindung R³-Me, R⁴-Me bzw. Me-R³-R⁴-Me zu. Man setzt die Organometallverbindung in der stöchiometrisch erforderlichen Menge oder in einem stöchiometrischen Überschuß ein.

Man setzt je Mol Oxaphosphorin der Formel (II) in der ersten Reaktionsstufe 1,0 bis 1,5, insbesondere 1,0 bis 1,3 Mol der Organometallverbindungen R³-Me oder R⁴-Me bzw. Me-R³-R⁴-Me zu und in der zweiten Reaktionsstufe setzt man je Mol Oxaphosphorin der Formel (II) 1,0 bis 1,5, insbesondere 1,0 bis 1,3 Mol der Organometallverbindungen R³-Me oder R⁴-Me zu.

Die Umsetzung erfolgt bedingt durch die Verwendung der wasserempfindlichen Organometallverbindungen, in Abwesenheit von Wasser. Es empfiehlt sich, die Reaktion unter einer Schutzgasatmosphäre, beispielsweise unter trockenem N₂ oder Argon, ablaufen zu lassen.

Man kann auch umgekehrt verfahren und die Lösung oder Suspension der Organometallverbindung vorlegen und das Oxaphosphorin bzw. die Lösung des Oxaphosphorins unter Rühren zudosieren.

Nach Abschluß der Reaktion kühlt man, falls eine hohe Reaktionstemperatur dies erforderlich machen sollte, das Reaktionsgemisch ab und setzt Wasser zu. Der Zusatz von Wasser ist erforderlich, um eventuell noch vorhandene Organometallverbindungen und die infolge der Reaktion gebildeten Produkte zu hydrolysieren.

Um die Phasentrennung zu begünstigen bzw. herbeizuführen kann man dem Reaktionsgemisch ein wasserunlösliches organisches Solvens zusetzen. Üblicherweise eignen sich Toluol, ortho-Xylol, meta-Xylol, para-Xylol, Gemische isomerer Xylole, Mesitylen, Ethylbenzol, Ester, die sich von einer Carbonsäure, insbesondere einer aliphatischen Carbonsäure mit 1 bis 8, insbesondere 1 bis 4 Kohlenstoffatomen und einem aliphatischen Alkohol mit 1 bis 8, insbesondere 1 bis 4 Kohlenstoffatomen ableiten, beispielsweise Ethyl-, Propyl- oder Butylacetat, als wasserunlösliches organisches Solvenz.

In einigen Fällen kann es nützlich sein, das Reaktionsgemisch oder die darin enthaltene wäßrige Phase auf einen pH-Wert ≦ 10, einzustellen. Dies begünstigt die Phasentrennung zusätzlich. Üblicherweise genügt es einen pH-Wert von 3 bis 9, insbesondere 4 bis 8, bevorzugt 5 bis 7 einzustellen. Die Phasen werden anschließend getrennt, die organische, in der Regel das Wertprodukt enthaltende Phase wird getrocknet und eingeengt, beispielsweise durch Abdestillieren des Lösungsmittel unter reduziertem Druck.

Die weitere Reinigung der Hydroxybiarylphosphane der Formel (I) erfolgt üblicherweise durch Kristallisation.

Das erfindungsgemäße Verfahren läßt sich kontinuierlich oder diskontinuierlich, besonders einfach diskontinuierlich ausführen. Es läßt sich bei Überdruck, Normaldruck oder Unterdruck, besonders einfach unter dem sich jeweils einstellenden Reaktionsdruck, durchführen.

Gegenstand der vorliegenden Erfindung sind ferner neue Hydroxybiarylphosphane der allgemeinen Formel I 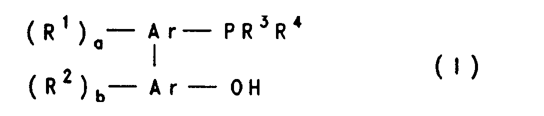 worin Ar-Ar für Biphenyl, 1-Phenylnaphthyl oder 1,1'-Binaphthyl, insbesondere für 1-Phenylnaphthyl steht, R¹ und R² gleich oder verschieden sind und für F, Cl, Alkyl oder Alkoxy mit jeweils 1 bis 8 Kohlenstoffatomen oder substituiertes oder unsubstituiertes Aryl stehen, a und b gleich oder verschieden sind und für eine ganze Zahl von 0 bis 4 stehen, die Ar-P- und die Ar-O-Bindung jeweils in ortho-Stellung zur Ar-Ar-Bindung angeordnet ist, R³ und R⁴ gleich oder verschieden sind und für Alkyl mit 1 bis 8 Kohlenstoffatomen, Cycloalkyl mit 4 bis 10 Kohlenstoffatomen, Alkaryl mit 7 bis 9 Kohlenstoffatomen, Ar³-(R⁵)ₙ, worin Ar³ ein Arylrest mit 6 bis 10 Kohlenstoffatomen, R⁵ Alkyl oder Alkoxy mit jeweils 1 bis 8 Kohlenstoffatomen, F, Cl, CF₃, Dialkylamino mit insgesamt 2 bis 8 Kohlenstoffatomen oder -CH₂N(Alkyl)₂ mit 1 bis 3 Kohlenstoffatomen je Alkyl ist und n eine ganze Zahl von 0 bis 5 darstellt, stehen, oder -R³-R⁴- eine Kette von 3 bis 5 Gliedern bildet, an der gegebenenfalls ein oder zwei aromatische Ringe oder Ringsysteme mit 6 bis 10 Kohlenstoffatomen anelliert sind, und mit dem P-Atom einen Ring mit 4 bis 6 Gliedern bildet, mit der Maßgabe, daß Ar³-(R⁵)ₙ nicht für Phenyl oder ein durch Halogen, Alkyl oder Alkoxy mit jeweils 1 bis 8 Kohlenstoffatomen substituiertes Phenyl steht, falls Ar-Ar Biphenyl oder 1,1'-Binaphthyl ist und R³ und R⁴ jeweils gleich sind.

Die Erfindung betrifft im besonderen Hydroxybiarylphosphane der Formel (I) worin Ar-Ar für Biphenyl, 1-Phenylnaphthyl oder 1,1-Binaphthyl, insbesondere für 1-Phenylnaphthyl steht, R¹ und R² gleich oder verschieden sind und für F, Cl, Alkyl oder Alkoxy mit jeweils 1 bis 4 Kohlenstoffatomen oder substituiertes oder unsubstituiertes Phenyl stehen, a und b gleich oder verschieden sind und für eine ganze Zahl von 0 bis 2, insbesondere 0 oder 1 stehen.

Die Erfindung betrifft ferner Hydroxybiarylphosphane der Formel (I), worin Ar-Ar für Biphenyl, 1-Phenylnaphthyl oder 1,1'-Binaphthyl, insbesondere für 1-Phenylnaphthyl steht, R³ und R⁴ gleich oder verschieden sind und für Alkyl mit 1 bis 4 Kohlenstoffatomen, Cycloalkyl mit 4 bis 6 Kohlenstoffatomen, Benzyl, Ar³-(R⁵), wobei Ar³ ein Arylrest mit 6 bis 10 Kohlenstoffatomen, R⁵ Alkyl oder Alkoxy mit jeweils 1 bis 4 Kohlenstoffatomen, F, Cl, CF₃, Dialkylamino mit insgesamt 2 bis 8 Kohlenstoffatomen oder -CH₂N(Alkyl)₂ mit 1 bis 3 Kohlenstoffatomen je Alkyl ist und n eine ganze Zahl von 0 bis 5 darstellt, stehen, mit der Maßgabe, daß Ar³-(R⁵)ₙ nicht für Phenyl oder ein durch Halogen, Alkyl oder Alkoxy mit jeweils 1 bis 8 Kohlenstoffatomen substituiertes Phenyl steht, falls Ar-Ar Biphenyl oder 1,1'-Binaphthyl ist und R³ und R⁴ jeweils gleich sind.

Für den Fall, daß Ar-Ar für 1-Phenylnaphthyl steht, zeichnen sich die neuen Hydroxybiarylphosphane dadurch aus, daß der Rest -PR³R⁴ entweder am Phenyring des 1-Phenylnaphthyls oder am Naphthylring des 1-Phenylnaphthyls angeordnet ist.

Ohne Anspruch auf Vollständigkeit zu erheben, seien als neue Verbindungen die folgenden Stoffe erwähnt:
2-Diphenylphosphino-1-(2-hydroxyphenyl)-naphthalin
2-Dicyclohexylphosphino-1-(2-hydroxyphenyl)-naphthalin
2-Bis-(4-fluorphenyl)phosphino**-**1-(2-hydroxyphenyl)-naphthalin
2-Bis-(2-methoxyphenyl)phosphino-1-(2-hydroxyphenyl)-naphthalin
2-Bis-(2-methylphenyl)phosphino**-**1-(2**-**hydroxyphenyl)-naphthalin
2-(4-Fluorphenyl-phenyl)phosphino-1-(2-hydroxyphenyl)-naphthalin
2-(2-Methoxyphenyl-phenyl)phosphino-1-(2-hydroxyphenyl)-naphthalin
1-[2-(Diphenylphosphino)phenyl]-2-hydroxynaphthalin
1-[2-(Dicyclohexylphosphino)phenyl]-2-hydroxynaphthalin
1-{2-[Bis-(4-fluorphenyl)phosphino]phenyl}-2-hydroxynaphthalin
1-[2-(4-Fluorphenyl-phenyl)phosphino-phenyl]-2-hydroxynaphthalin
1-[2-(2-Methoxyphenyl-phenyl)phosphino-phenyl]-2-hydroxynaphthalin
2-(Diisopropyl)phosphino-1-(2-hydroxyphenyl)-naphthalin
2-[Bis-(4-N,N-dimethylaminophenyl)phosphino]-1-(2-hydroxyphenyl)-naphthalin
2-[Bis-(4-N,N-diisopropylaminomethylphenyl)phosphino]-1-(2-hydroxyphenyl)-naphthalin
2-{Bis-[3,5-bis-(trifluormethyl)phenyl]phosphino}-1-(2-hydroxyphenyl)-naphthalin
1-[2-(Diisopropylphosphino)phenyl]-2-hydroxynaphthalin
1-{2-[Bis-(4-N,N-dimethylaminophenyl)phosphino]phenyl}-2-hydroxynaphthalin
1-{2-[Bis-(4-N,N-diisopropylaminomethylphenyl)phosphino]phenyl}-2-hydroxynaphthalin
1-{2-(Bis-{3,5-bis-(trifluormethyl)phenyl]phosphino}phenyl}-2-hydroxynaphthalin
2'-Hydroxy-2-[2-methoxyphenyl(phenyl)phosphino]-biphenyl
2'-Hydroxy-2-[isopropyl(phenyl)phosphino]-biphenyl
2'-Hydroxy-2-[cyclohexyl(phenyl)phosphino]-biphenyl
2-{Bis-[3,5-bis-(trifluormethyl)phenyl]phosphino}-2'-hydroxy-biphenyl
2-Diisopropylphosphino-2'-hydroxy-biphenyl
2-[Bis-(4-N,N-dimethylaminophenyl)phosphino]-2'-hydroxy-biphenyl
2-[Bis-(4-N,N-diisopropylaminomethylphenyl)phosphino]-2'-hydroxy-biphenyl
2-Diphenylphosphino-2'-hydroxy-3'-phenyl-biphenyl
2'-Hydroxy-2-[2-methoxyphenyl(phenyl)phosphino]-1,1'-biphenyl
2-{Bis-[3,5-bis-(trifluormethyl)phenyl]phosphino}-2'-hydroxy-1,1'-binaphthyl
2-Diisopropylphosphino-2'-hydroxy-1,1'-binaphthyl
2-[Bis-(4-N,N-dimethylaminophenyl)phosphino]-2'-hydroxy-1,1'-binaphthyl
2-[Bis**-**(4**-**N,N**-**diisopropylaminomethylphenyl)phosphino]-2'-hydroxy-1,1'-binaphthyl
2'-Hydroxy-2-[(isopropyl-phenyl)phosphino]-1,1'-binaphthyl
2'-Hydroxy-2-[(cyclohexyl-phenyl)phosphino]-1,1'-binaphthyl

Die nachfolgenden Beispiele beschreiben die Erfindung näher, ohne sie darauf zu beschränken.

### Experimenteller Teil

### Beispiel 1

### Herstellung von 2'-Hydroxy-2-diphenylphosphino-biphenyl:

In einer Argonatmosphäre werden unter Rühren 12,3 g (52,4 mmol) 6-Chlor-6H-dibenz[c,e][1,2]oxaphosphorin in 80 ml trockenem Tetrahydrofuran vorgelegt und 66 ml einer 2M-Lösung von Phenylmagnesiumbromid in Tetrahydrofuran zugetropft. Dabei steigt die Temperatur von 25 auf 40°C. Man rührt anschließend 4 Stunden bei 65°C, kühlt auf Raumtemperatur ab und versetzt mit 100 ml Wasser und 100 ml Ethylacetat. Man neutralisiert mit verdünnter Salzsäure, trennt die organische Phase ab, trocknet mit Natriumsulfat und engt unter reduziertem Druck ein. Man erhält 19,0 g farbloses Öl. Durch Kristallisation mit Methanol/Wasser 10:1 erhält man 18,1 g (98 %) farblose Kristalle mit Schmelzpunkt 123 bis 124°C.
³¹P**-**NMR (CDCl₃): δ = -11,7 ppm

### Beispiel 2

### 2'-Hydroxy-2-[bis-(3-fluorphenyl)phosphino]-biphenyl:

In einer Argonatmosphäre werden unter Rühren 16,4 g (70 mmol) 6-Chlor-6H-dibenz[c,e][1,2]oxaphosphorin in 80 ml trockenem Tetrahydrofuran vorgelegt und eine 2M-Lösung von 140 mmol 3-Fluorphenylmagnesiumbromid in Tetrahydrofuran zugetropft. Dabei steigt die Temperatur von 25 auf 45°C. Man rührt anschließend 12 Stunden bei 65°C. Man kühlt auf Raumtemperatur ab und versetzt mit 100 ml Wasser und 100 ml Ethylacetat. Man neutralisiert mit verdünnter Salzsäure, trennt die organische Phase ab, trocknet mit Natriumsulfat und engt unter reduziertem Druck ein. Man erhält 25,8 farbloses Öl. Durch Kristallisation mit Methanol/Wasser 10:1 erhält man 19,8 g (71 %) farblose Kristalle mit Schmelzpunkt 161 bis 163°C.
³¹P-NMR (CDCl₃): δ = -11,0 ppm

### Beispiel 3

### Herstellung von 2'-Hydroxy-2-[bis-(1-naphthyl)phosphino]-biphenyl:

In einer Argonatmosphäre werden unter Rühren 14,1 g (60 mmol) 6-Chlor-6H-dibenz[c,e][1,2]oxaphosphorin in 50 ml trockenem o-Xylol vorgelegt und eine Lösung von 150 mmol 1-Naphthylmagnesiumbromid in Tetrahydrofuran zugetropft. Dabei steigt die Temperatur von 25 auf 45°C. Man rührt anschließend 3 Stunden bei 80°C und 5 Stunden 120°C. Man kühlt auf Raumtemperatur ab und versetzt mit 100 ml Wasser. Man neutralisiert mit verdünnter Salzsäure, trennt die organische Phase ab, trocknet mit Natriumsulfat und engt unter reduziertem Druck ein. Man erhält 19,0 g farbloses Öl. Durch Kristallisation mit Methanol/Wasser 10:1 erhält man 22,8 g (89 %) farblose Kristalle mit Schmelzpunkt 255 bis 256°C.
³¹P-NMR (CDCl₃): δ = -30,3 ppm

### Beispiel 4

### Herstellung von 2'-Hydroxy-2-[(2-methoxyphenyl-phenyl)phosphino]-biphenyl:

In einer Argonatmosphäre werden unter Rühren 23,5 g (100 mmol) 6-Chlor-6H-dibenz[c,e][1,2]oxaphosphorin in 50 ml trockenem Tetrahydrofuran vorgelegt und eine Lösung von 100 mmol 2-Methoxyphenylmagnesiumbromid in Tetrahydrofuran bei -10°C zugetropft. Man erwärmt langsam auf 25°C und rührt 3 Stunden. Man tropft eine Lösung von 100 mmol Phenylmagnesiumbromid in 150 ml Tetrahydrofuran und rührt anschließend 5 Stunden bei 65°C. Man kühlt auf Raumtemperatur ab und versetzt mit 100 ml Wasser. Man neutralisiert mit verdünnter Salzsäure, trennt die organische Phase ab, trocknet mit Natriumsulfat und engt unter reduziertem Druck ein. Man erhält 38,0 g farbloses Öl. Durch Kristallisation mit Methanol/Wasser 10:1 erhält man 32,7 g (86 %) farblose Kristalle mit Schmelzpunkt 137 bis 139°C.
³¹P-NMR (CDCl₃): δ = -22,0 und -23,2 ppm (Diastereomerenverhältnis 30:70)

### Beispiel 5

### Herstellung von 2'-Hydroxy-2-[(2-tolyl-phenyl)phosphino]-biphenyl:

In einer Argonatmosphäre werden unter Rühren 16,4 g (70 mmol) 6-Chlor-6H-dibenz[c,e][1,2]oxaphosphorin in 50 ml trockenem o-Xylol vorgelegt und eine Lösung von 70 mmol 2-Tolylmagnesiumbromid in Tetrahydrofuran bei -10°C zugetropft. Man erwärmt langsam auf 25°C und rührt 3 Stunden. Man tropft eine Lösung von 70 mmol Phenylmagnesiumbromid in 50 ml Diethylether zu und erwärmt anschließend auf 65°C, wobei der Diethylether abdestilliert wird. Man rührt noch 2 Stunden bei 120°C kühlt auf Raumtemperatur ab und versetzt mit 100 ml Wasser. Man neutralisiert mit verdünnter Salzsäure, trennt die organische Phase ab, trocknet mit Natriumsulfat und engt unter reduziertem Druck ein. Man erhält 25,8 g farblose Kristalle. Durch Umkristallisation aus Methanol/Wasser 10:1 erhält man 20,4 g (79 %) farblose Kristalle mit Schmelzpunkt 98 bis 101°C.
³¹P-NMR (CDCl₃): δ = -19,7 und -20,2 ppm (Diastereomerenverhältnis 50:50)

### Beispiel 6

### Herstellung von 1-(2-Hydroxyphenyl)-2-diphenylphosphino-naphthalin:

In einer Argonatmosphäre werden unter Rühren 50,8 g (178 mmol) 6-Chlor-6H-benzo[c]naphth[2,1-c][1,2]oxaphosphorin in 250 ml trockenem o-Xylol vorgelegt und 390 ml einer 2M-Lösung von Phenylmagnesiumbromid in Diethylether zugetropft. Dabei steigt die Temperatur von 25 auf 40°C. Man erwärmt anschließend langsam auf 100°C, wobei der Diethylether abdestilliert, und rührt 3 Stunden. Man kühlt auf Raumtemperatur ab und versetzt mit 100 ml Wasser und 100 ml Ethylacetat. Man neutralisiert mit verdünnter Salzsäure, trennt die organische Phase ab, trocknet mit Natriumsulfat und engt unter reduziertem Druck ein. Man erhält 70 g farbloses Öl. Durch Kristallisation mit Methanol erhält man 63,8 g (89 %) farblose Kristalle mit Schmelzpunkt 104 bis 106°C.
³¹P-NMR (CDCl₃): δ = -12,0 ppm

### Beispiel 7

### Herstellung von 1-(2-Diphenylphosphinophenyl)-2-hydroxy-naphthalin

In einer Argonatmosphäre werden unter Rühren 9,5 g (33,5 mmol) 5-Chlor-5H-benzo[c]naphth[1,2-e][1,2]oxaphosphorin in 50 ml trockenem o-Xylol vorgelegt und 28 ml einer Lösung von 84 mmol Phenylmagnesiumbromid in Diethylether zugetropft. Dabei steigt die Temperatur von 25 auf 40°C. Man erwärmt anschließend langsam auf 100°C, wobei der Diethylether abdestilliert, und rührt 3 Stunden. Man kühlt auf Raumtemperatur ab und versetzt mit 100 ml Wasser und 100 ml Ethylacetat. Man neutralisiert mit verdünnter Salzsäure, trennt die organische Phase ab, trocknet mit Natriumsulfat und engt unter reduziertem Druck ein. Man erhält 13,5 g farbloses Öl. Durch Kristallisation mit Methanol erhält man 9,5 g (70 %) farblose Kristalle mit Schmelzpunkt 135 bis 138°C.
³¹P-NMR (CDCl₃): δ = -12,7 ppm

### Beispiel 8

### Herstellung von 2-[Bis-(3-fluorphenyl)phosphino]-1-(2-hydroxyphenyl)-naphthalin:

In einer Argonatmosphäre werden unter Rühren 8,5 g (30 mmol) 5-Chlor-5H-benzo[c]naphth[1,2-e][1,2]-oxaphosphorin in 50 ml trockenem o-Xylol bei 80°C vorgelegt und eine Lösung von 70 mmol 3-Fluorphenylmagnesiumbromid in 80 ml o-Xylol zugetropft. Dabei steigt die Temperatur von 80 auf 90°C. Man rührt anschließend 1 Stunde bei 110°C. Man kühlt auf Raumtemperatur ab und versetzt mit 100 ml Wasser. Man neutralisiert mit verdünnter Salzsäure, trennt die organische Phase, trocknet mit Natriumsulfat und engt unter reduziertem Druck ein. Man erhält 19,0 g farbloses Öl.
³¹P-NMR (CDCl₃): δ = -3,8 ppm

### Beispiel 9

### Herstellung von 2-(Dibenzophosphol-9-yl)-2'-hydroxy-biphenyl:

Zu einer Lösung von 11,4 g (36,54 mmol) 2,2'-Dibrombiphenyl in 60 ml Diethylether werden 46 ml einer 1,6 M-Lösung von Butyllithium in Hexan getropft. Anschließend rührt man 24 Stunden bei 20°C. Dazu tropft man dann bei 0°C eine Lösung von 8,6 g (36,5 mmol) 6-Chlor-6H-dibenz[c,e][1,2]oxaphosphorin in 30 ml o-Xylol. Anschließend rührt man noch 2 Stunden bei 25°C und 3 Stunden bei 50°C. Man kühlt auf Raumtemperatur ab und versetzt vorsichtig mit 100 ml Wasser. Man neutralisiert mit verdünnter Salzsäure, trennt die organische Phase ab, trocknet mit Natriumsulfat und engt unter reduziertem Druck ein. Durch Kristallisation des Rückstands mit 2-Butanon erhält man 11,8 g (90 %) farblose Kristalle mit Schmelzpunkt 152 bis 155°C.
³¹P-NMR (CDCl₃): δ = -17,8 ppm

### Beispiel 10

### 2-{Bis-[3,5-bis-(trifluormethyl)phenyl]phosphino}-2'-hydroxy-biphenyl:

In einer Argonatmosphäre werden unter Rühren 23,5 g (0,1 mol) 6-Chlor-6H-dibenz[c,e][1,2]oxaphosphorin in 200 ml trockenem o-Xylol bei 80°C vorgelegt und eine Lösung von 0,23 mol 3,5-Di-(trifluormethyl)phenylmagnesiumbromid in 300 ml Tetrahydrofuran zugetropft. Dabei wird Tetrahydrofuran abdestilliert und die Temperatur langsam auf 120°C erhöht. Man rührt anschließend 3 Stunden bei 120°C, kühlt auf Raumtemperatur ab und versetzt mit 100 ml Wasser. Man neutralisiert mit verdünnter Salzsäure, trennt die organische Phase ab, trocknet mit Natriumsulfat und engt unter reduziertem Druck ein. Man erhält 61,0 g schwarzen Feststoff. Durch Umkristallisation unter Zusatz von Aktivkohle aus Heptan und anschließender Heißfiltration erhält man 37,2 g (59 %) blaßgelbe Kristalle mit Schmelzpunkt 120 bis 121°C.
³¹P-NMR (CDCl₃): δ = -8,4 ppm

### Beispiel 11

### Herstellung von 2-Diisopropylphosphino-2'-hydroxy-biphenyl:

In einer Argonatmosphäre werden unter Rühren 46,9 g (0,2 mol) 6-Chlor-6H-dibenz[c,e][1,2]oxaphosphorin in 250 ml trockenem o-Xylol bei 80°C vorgelegt und eine Lösung von 0,45 mol Isopropylmagnesiumbromid in 300 ml Tetrahydrofuran zugetropft. Dabei wird Tetrahydrofuran abdestilliert und die Temperatur langsam auf 120°C erhöht. Man rührt anschließend 3 Stunden bei 120°C, kühlt auf Raumtemperatur ab und versetzt mit 100 ml Wasser. Man neutralisiert mit konzentrierter Schwefelsäure, erwärmt auf 80°C und trennt die organische Phase ab. Beim Abkühlen kristallisieren aus der wäßrigen Phase 51,7 g (90 %) farbloser Feststoff mit Schmelzpunkt 82 bis 84°C. Dieser Feststoff wird mit 100 ml 2n Natronlauge versetzt und mit Toluol extrahiert. Die Toluol-Phase wird abgetrennt, mit Wasser gewaschen, anschließend mit Natriumsulfat getrocknet und im Vakuum eingeengt. Man erhält 38,9 g (68 %) farbloses öl.
³¹P-NMR (CDCl₃): δ = 0,3 ppm

### Beispiel 12

### Herstellung von 2-[Bis-(4-N,N-dimethylaminophenyl)phosphino]-2'-hydroxybiphenyl:

In einer Argonatmosphäre wird unter Rühren eine Lösung von 300 mmol 4-N,N-Dimethylaminophenylmagnesiumbromid in 150 ml Tetrahydrofuran vorgelegt und es werden 30,5 g (130 mmol) 6-Chlor-6H-dibenz[c,e][1,2]oxaphosphorin in 200 ml trockenem o-Xylol bei 80°C zugetropft. Dabei steigt die Temperatur von 80 auf 90°C, wobei Tetrahydrofuran abdestilliert wird. Man rührt anschließend 1 Stunde bei 110°C. Man kühlt auf Raumtemperatur ab und versetzt mit 100 ml Wasser. Man neutralisiert mit verdünnter Salzsäure, trennt die organische Phase ab, trocknet mit Natriumsulfat und engt unter reduziertem Druck ein. Nach Kristallisation mit Methanol erhält man 48,0 g (84 %) farblose Kristalle mit Schmelzpunkt 177 bis 178°C.
³¹P-NMR (CDCl₃): δ = -15,6 ppm

### Beispiel 13

### Herstellung von 2-Diphenylphosphino-2'-hydroxy-3'-phenyl-biphenyl:

In einer Argonatmosphäre werden unter Rühren 23,5 g (0,1 mol) 6-Chlor-6H-4-phenyl-dibenz[c,e][1,2]oxaphosphorin in 120 ml trockenem o-Xylol bei 80°C vorgelegt und eine Lösung von 0,25 mol Phenylmagnesiumbromid in 100 ml Diethylether zugetropft. Dabei wird Diethylether abdestilliert und die Temperatur langsam auf 120°C erhöht. Man rührt anschließend 3 Stunden bei 120°C, kühlt auf Raumtemperatur ab und versetzt mit 100 ml Wasser. Man neutralisiert mit verdünnter Salzsäure, trennt die organische Phase ab, trocknet mit Natriumsulfat und engt unter reduziertem Druck ein. Man erhält 40,0 g farbloses zähes Öl.
³¹P-NMR (CDCl₃): δ = -11,0 ppm

### Beispiel 14

### Herstellung von 2-[Bis-(4-N,N-diisopropylaminomethylphenyl)phosphino]-2'-hydroxy-biphenyl:

In einer Argonatmosphäre wird unter Rühren eine Lösung von 300 mmol 4-(N,N-Diisopropylaminomethyl)phenyl-magnesiumbromid in 150 ml Tetrahydrofuran (THF) vorgelegt und 30,5 g (130 mmol) 6-Chlor-6H-dibenz[c,e][1,2]oxaphosphorin in 200 ml trockenem o-Xylol bei 80°C zugetropft. Die Temperatur steigt von 80 auf 90°C, dabei wird Tetrahydrofuran abdestilliert. Man rührt anschließend 5 Stunden bei 120°C. Man kühlt auf Raumtemperatur ab und versetzt mit 100 ml Wasser. Man neutralisiert mit verdünnter Salzsäure, trennt die organische Phase ab, trocknet mit Natriumsulfat und engt im Vakuum ein. Nach Kristallisation mit Methanol erhält man 63,3 g (84 %) farblose Kristalle.
³¹P-NMR (CDCl₃): δ = -12,7 ppm

### Beispiel 15

### Herstellung von 2'-{Bis-[3,5-bis-(trifluormethyl)phenyl]phosphino-phenyl}-2-hydroxynaphthalin:

In einer Argonatmosphäre werden unter Rühren 6,3 g (22 mmol) 5-Chlor-5H-benzo[c]naphth[1,2-e][1,2]oxaphosphorin in 100 ml trockenem o-Xylol bei 80°C vorgelegt und dazu wird eine Lösung von 0,86 mmol 3,5-Bis-(trifluormethyl)phenylmagnesiumbromid in 120 ml Tetrahydrofuran zugetropft. Dabei wird Tetrahydrofuran abdestilliert und die Temperatur langsam auf 120°C erhöht. Man rührt schließlich 4 Stunden bei 120°C, kühlt auf Raumtemperatur ab und versetzt mit 100 ml Wasser. Man neutralisiert mit verdünnter Salzsäure, trennt die organische Phase ab, trocknet mit Natriumsulfat und engt im Vakuum ein. Man erhält 15,9 g schwarzen Feststoff. Durch Umkristallisation unter Zusatz von Aktivkohle aus Heptan und anschließender Heißfiltration erhält man 9,5 g (64 %) blaßgelbes zähes Öl.
³¹P-NMR (CDCl₃): δ = -9,7 ppm

### Beispiel 16

### Herstellung von 2'-[Bis-(di-3,5-fluorphenyl)phosphino-phenyl]-2-hydroxynaphthalin:

In einer Argonatmosphäre werden unter Rühren 14,0 g (0,049 mol) 5-Chlor-5H-benzo[c]naphth[1,2-e][1,2]oxaphosphorin in 100 ml trockenem o-Xylol bei 80°C vorgelegt und dazu wird eine Lösung von 0,15 mol 3,5-Difluorphenylmagnesiumbromid in 100 ml Tetrahydrofuran zugetropft. Dabei wird Tetrahydrofuran abdestilliert und die Temperatur langsam auf 120°C erhöht. Man rührt anschließend 3 Stunden bei 120°C, kühlt auf Raumtemperatur ab und versetzt mit 100 ml Wasser. Man neutralisiert mit verdünnter Salzsäure, trennt die organische Phase ab, trocknet mit Natriumsulfat und engt im Vakuum ein. Man erhält 23,3 g braunes Öl. Durch Säulenchromatographie auf Kieselgel mit Ethylacetat/n-Heptan (2:1) als Laufmittel erhält man 13,7 g (59 %) farblose Kristalle.
³¹P-NMR (CDCl₃): δ = -9,0 ppm

### Beispiel 17

### Herstellung von 2-{Bis-[3,5-bis-(trifluormethyl)phenyl]phosphino}-2'-hydroxy-3'-phenyl-biphenyl:

In einer Argonatmosphäre werden unter Rühren 10,0 g (0,032 mol) 6-Chlor-6H-4-phenyl-dibenz[c,e][1,2]oxaphosphorin in 100 ml trockenem o-Xylol bei 80°C vorgelegt und dazu wird eine Lösung von 0,086 mol 3,5-Bis-(trifluormethyl)phenyl-magnesiumbromid in 100 ml Tetrahydrofuran zugetropft. Dabei wird Tetrahydrofuran abdestilliert und die Temperatur langsam auf 120°C erhöht. Man rührt anschließend 3 Stunden bei 120°C, kühlt auf Raumtemperatur ab und versetzt mit 100 ml Wasser. Man neutralisiert mit verdünnter Salzsäure, trennt die organische Phase ab, trocknet mit Natriumsulfat und engt im Vakuum ein. Man erhält 22,5 g braunes zähes Öl.
³¹P-NMR (CDCl₃): δ = -8,0 ppm

### Beispiel 18

### Herstellung von 2-[Bis-(di-3,5-fluorphenyl)phosphino]-2'-hydroxy-3'-phenylbiphenyl:

In einer Argonatmosphäre werden unter Rühren 11,7 g (0,05 mol) 6-Chlor-6H-4-phenyl-dibenz[c,e][1,2]oxaphosphorin in 120 ml trockenem o-Xylol bei 80°C vorgelegt und dazu wird eine Lösung von 0,125 mol 3,5-Difluorphenylmagnesiumbromid in 100 ml Tetrahydrofuran zugetropft. Dabei wird Tetrahydrofuran abdestilliert und die Temperatur langsam auf 120°C erhöht. Man rührt anschließend 3 Stunden bei 120°C, kühlt auf Raumtemperatur ab und versetzt mit 100 ml Wasser. Man neutralisiert mit verdünnter Salzsäure, trennt die organische Phase ab, trocknet mit Natriumsulfat und engt im Vakuum ein. Man erhält 21,3 g farbloses zähes Öl.
³¹P-NMR (CDCl₃): δ = -7,5 ppm

## Patentansprüche

1. Verfahren zur Herstellung von Hydroxybiarylphosphanen der allgemeinen Formel 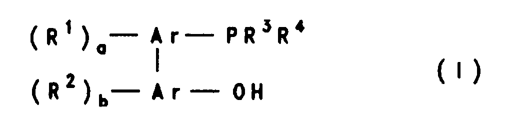 worin R¹, R², a, b, Ar-Ar, R³ und R⁴ die nachfolgende Bedeutung haben, dadurch gekennzeichnet, daß man ein Oxaphosphorin der allgemeinen Formel 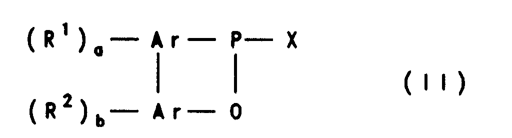 worin Ar-Ar für Biphenyl, 1-Phenylnaphthyl oder 1,1'-Binaphthyl steht, R¹ und R² gleich oder verschieden sind und für F, Cl, Alkyl oder Alkoxy mit jeweils 1 bis 8 Kohlenstoffatomen oder substituiertes oder unsubstituiertes Aryl stehen, a und b gleich oder verschieden sind und für eine ganze Zahl von 0 bis 4 stehen, X jeweils für Cl oder Br steht und die Ar-P und die Ar-O-Bindung jeweils in ortho-Stellung zur Ar-Ar-Bindung angeordnet ist, mit einer Verbindung R³-Me und R⁴-Me, worin R³ und R⁴ gleich oder verschieden sind und für Alkyl mit 1 bis 8 Kohlenstoffatomen, Cycloalkyl mit 4 bis 10 Kohlenstoffatomen, Alkaryl mit 7 bis 9 Kohlenstoffatomen, Ar³-(R⁵)ₙ, wobei Ar³ ein Arylrest mit 6 bis 10 Kohlenstoffatomen, R⁵ Alkyl oder Alkoxy mit jeweils 1 bis 8 Kohlenstoffatomen, F, Cl, CF₃, Dialkylamino mit insgesamt 2 bis 8 Kohlenstoffatomen oder -CH₂N(Alkyl)₂, mit 1 bis 3 Kohlenstoffatomen je Alkyl ist und n eine ganze Zahl von 0 bis 5 darstellt, stehen, oder mit einer Verbindung Me-R³-R⁴-Me, worin -R³-R⁴- eine Kette von 3 bis 5 Gliedern bildet, an der gegebenenfalls ein oder zwei aromatische Ringe oder Ringsysteme mit 6 bis 10 Kohlenstoffatomen anelliert sind, Me für ein einwertiges Metall oder für ein Äquivalent eines mehrwertigen Metalles steht, in Anwesenheit eines Lösungsmittels bei einer Temperatur von -40 bis 160°C umsetzt, anschließend mit Wasser und gegebenenfalls einem wasserunlöslichen organischen Solvens versetzt und die wäßrige Phase von der organischen Phase trennt.

2. Verfahren nach Anspruch 1, dadurch gekennzeichnet, daß man ein Oxaphosphorin der Formel (II) einsetzt, worin R¹ und R² gleich oder verschieden sind und für F, Cl, Alkyl oder Alkoxy mit jeweils 1 bis 4 Kohlenstoffatomen oder substituiertes oder unsubstituiertes Phenyl stehen, a und b gleich oder verschieden sind und für eine ganze Zahl von 0 bis 2 stehen.

3. Verfahren nach Anspruch 1 oder 2, dadurch gekennzeichnet, daß man ein Oxaphosphorin der allgemeinen Formel (II) einsetzt, worin R¹ und R² gleich oder verschieden sind und für F, Cl, Alkyl oder Alkoxy mit jeweils 1 bis 4 Kohlenstoffatomen stehen, a und b gleich oder verschieden sind und für 0 oder 1 stehen.

4. Verfahren nach einem oder mehreren der Ansprüche 1 bis 3, dadurch gekennzeichnet, daß man ein Oxaphosphorin der Formel (II) einsetzt, worin Ar-Ar für 1-Phenylnaphthyl steht und die Ar-P-Bindung am Phenylring des 1-Phenylnaphthyls oder am Naphthylring des 1-Phenylnaphthyls angeordnet ist.

5. Verfahren nach einem oder mehreren der Ansprüche 1 bis 4, dadurch gekennzeichnet, daß man eine Verbindung R³-Me oder R⁴-Me einsetzt, worin R³ und R⁴ gleich oder verschieden sind und für Alkyl mit 1 bis 4 Kohlenstoffatomen, Cycloalkyl mit 4 bis 6 Kohlenstoffatomen, Benzyl, Ar³-(R⁵)ₙ, wobei Ar³ ein Arylrest mit 6 bis 10 Kohlenstoffatomen und R⁵ H, Alkyl oder Alkoxy mit jeweils 1 bis 4 Kohlenstoffatomen, F, Cl, CF₃, Dialkylamino mit insgesamt 2 bis 8 Kohlenstoffatomen oder -CH₂N(Alkyl)₂ mit 1 bis 3 Kohlenstoffatomen je Alkyl ist und n eine ganze Zahl von 0 bis 5 darstellt, stehen.

6. Verfahren nach einem oder mehreren der Ansprüche 1 bis 5, dadurch gekennzeichnet, daß man eine Verbindung R³-Me und R⁴-Me einsetzt, worin Me für MgX, wobei X für Chlor, Brom oder Jod steht, oder für ein Alkalimetall steht.

7. Verfahren nach einem oder mehreren der Ansprüche 1 bis 6, dadurch gekennzeichnet, daß man eine Verbindung R³-Me und R⁴-Me einsetzt, worin Me für MgX, wobei X für Chlor oder Brom steht, oder für Lithium steht.

8. Verfahren nach einem oder mehreren der Ansprüche 1 bis 7, dadurch gekennzeichnet, daß man als Lösungsmittel ein dipolar aprotisches oder unpolares Lösungsmittel oder eine Mischung dieser Lösungsmittel einsetzt.

9. Verfahren nach einem oder mehreren der Ansprüche 1 bis 8, dadurch gekennzeichnet, daß man als Lösungsmittel Tetrahydrofuran, Dioxan, ortho-Xylol, meta-Xylol, para-Xylol, ein Gemisch isomerer Xylole, Toluol, Mesitylen, Ethylbenzol oder Gemische dieser Lösungsmittel als Lösungsmittel einsetzt.

10. Verfahren nach einem oder mehreren der Ansprüche 1 bis 9, dadurch gekennzeichnet, daß man die Umsetzung bei einer Temperatur von 40 bis 160°C, bevorzugt 60 bis 120°C durchführt.

11. Verfahren nach einem oder mehreren der Ansprüche 1 bis 10, dadurch gekennzeichnet, daß man die Umsetzung in einer ersten Stufe bei -40 bis 80, insbesondere -20 bis 60, bevorzugt -10 bis 50°C und anschließend in einer zweiten Stufe bei 20 bis 160°C, insbesondere 40 bis 140, bevorzugt 60 bis 120°C durchführt.

12. Verfahren nach einem oder mehreren der Ansprüche 1 bis 11, dadurch gekennzeichnet, daß man Toluol, ortho-Xylol, meta-Xylol, para-Xylol, Gemische isomerer Xylole, Mesitylen, Ethylbenzol oder einen Ester einer aliphatischen Carbonsäure mit 1 bis 4 Kohlenstoffatomen und eines aliphatischen Alkohols mit 1 bis 4 Kohlenstoffatomen oder Gemische dieser Lösungsmittel als wasserunlösliches organisches Solvens einsetzt.

13. Verfahren nach einem oder mehreren der Ansprüche 1 bis 12, dadurch gekennzeichnet, daß man die wäßrige Phase auf einen pH-Wert von 3 bis 9, insbesondere 4 bis 8, bevorzugt 5 bis 7 einstellt.

14. Hydroxybiarylphosphane der allgemeinen Formel I 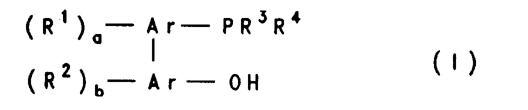 worin Ar-Ar für Biphenyl, 1-Phenylnaphthyl oder 1,1'-Binaphthyl steht, R¹ und R² gleich oder verschieden sind und für F, Cl, Alkyl oder Alkoxy mit jeweils 1 bis 8 Kohlenstoffatomen oder substituiertes oder unsubstituiertes Aryl stehen, a und b gleich oder verschieden sind und für eine ganze Zahl von 0 bis 4 stehen, die Ar-P- und die Ar-O-Bindung jeweils in ortho-Stellung zur Ar-Ar-Bindung angeordnet ist, R³ und R⁴ gleich oder verschieden sind und für Alkyl mit 1 bis 8 Kohlenstoffatomen, Cycloalkyl mit 4 bis 10 Kohlenstoffatomen, Alkaryl mit 7 bis 9 Kohlenstoffatomen, Ar³-(R⁵)ₙ, worin Ar³ ein Arylrest mit 6 bis 10 Kohlenstoffatomen, R⁵ Alkyl oder Alkoxy mit jeweils 1 bis 8 Kohlenstoffatomen, F, Cl, CF₃, Dialkylamino mit insgesamt 2 bis 8 Kohlenstoffatomen oder -CH₂N(Alkyl)₂ mit 1 bis 3 Kohlenstoffatomen je Alkyl ist und n eine ganze Zahl von 0 bis 5 darstellt, stehen oder -R³-R⁴-eine Kette von 3 bis 5 Gliedern bildet, an der gegebenenfalls ein oder zwei aromatische Ringe oder Ringsysteme mit 6 bis 10 Kohlenstoffatomen anelliert sind, und mit dem P-Atom einen Ring mit 4 bis 6 Gliedern bildet, mit der Maßgabe, daß Ar³-(R⁵)ₙ nicht für Phenyl oder ein durch Halogen, Alkyl oder Alkoxy mit jeweils 1 bis 8 Kohlenstoffatomen substituiertes Phenyl steht, falls Ar-Ar Biphenyl oder 1,1'-Binaphthyl ist und R³ und R⁴ jeweils gleich sind.

15. Hydroxybiarylphosphane nach Anspruch 14, dadurch gekennzeichnet, daß R¹ und R² gleich oder verschieden sind und für F, Cl, Alkyl oder Alkoxy mit jeweils 1 bis 4 Kohlenstoffatomen oder substituiertes oder unsubstituiertes Phenyl stehen, a und b gleich oder verschieden sind und für eine ganze Zahl von 0 bis 2 stehen.

16. Hydroxybiarylphosphane nach Anspruch 14 oder 15, dadurch gekennzeichnet, daß R³ und R⁴ gleich oder verschieden sind und für Alkyl mit 1 bis 4 Kohlenstoffatomen, Cycloalkyl mit 4 bis 6 Kohlenstoffatomen, Benzyl, Ar³-(R⁵), wobei Ar³ ein Arylrest mit 6 bis 10 Kohlenstoffatomen, R⁵ Alkyl oder Alkoxy mit jeweils 1 bis 4 Kohlenstoffatomen, F, Cl, CF₃, Dialkylamino mit insgesamt 2 bis 8 Kohlenstoffatomen oder -CH₂N(Alkyl)₂ mit 1 bis 3 Kohlenstoffatomen je Alkyl ist und n eine ganze Zahl von 0 bis 5 darstellt, stehen, mit der Maßgabe, daß Ar³-(R⁵)ₙ nicht für Phenyl oder ein durch Halogen, Alkyl oder Alkoxy mit jeweils 1 bis 8 Kohlenstoffatomen substituiertes Phenyl steht, falls Ar-Ar Biphenyl oder 1,1'-Binaphthyl ist und R³ und R⁴ jeweils gleich sind.

17. Hydroxybiarylphosphane nach einem oder mehreren der Ansprüche 14 bis 16, dadurch gekennzeichnet, daß der Rest -PR³R⁴ am Phenylring des 1-Phenylnaphthyls angeordnet ist.

18. Hydroxybiarylphosphane nach einem oder mehreren der Ansprüche 14 bis 16, dadurch gekennzeichnet, daß der Rest -PR³R⁴ am Naphthylring des 1-Phenylnaphthyls angeordnet ist.
